# EUROPEAN PATENT APPLICATION

(11) **EP 4 763 094 A1**
(43) Date of publication of application: **24.06.2026**
(21) Application number: 25224498.3
(22) Date of filing: 17.12.2025
(51) Int. Cl.: A61B 8/08, A61B 8/12, A61B 8/00

(54) **INTRA-PROCEDURE AUTOMATIC PERICARDIAL FLUID MONITORING BY INTRACARDIAC ECHOGRAPHY (ICE)**

(30) Priority: 18.12.2024 US 202418986035
(71) Applicant: Biosense Webster (Israel) Ltd., 2066717 Yokneam (IL)
(72) Inventor: KLEMM, Ofer, 2066717 Yokneam (IL)
(74) Representative: Carpmaels & Ransford LLP

(57) **Abstract**

A medical system includes an ultrasound probe and a processor. The ultrasound probe is configured for insertion into the heart of a body and comprises (a) an ultrasound transducer array configured to generate ultrasound images of a portion of the heart and (b) a sensor configured to produce signals indicative of an orientation of the ultrasound transducer array inside the heart. The processor is configured to (i) select, using the signals produced by the sensor, a sequence of two or more of the ultrasound images that are acquired from a given orientation of the ultrasound transducer array, wherein the ultrasound images image at least part of a pericardial space of the heart, (ii) estimate changes in Pericardial Effusion (PE) in the pericardial space, by analyzing the selected sequence of the ultrasound images, and (iii) initiate a responsive action when the changes meet a defined condition.

## Description

### FIELD OF THE DISCLOSURE

The present disclosure relates generally to imaging of human anatomy and particularly to real-time monitoring of cardiac anatomy using an ultrasound (US) probe.

### BACKGROUND OF THE DISCLOSURE

Pericardial Effusion (PE) is a common finding in cardiac catheter procedures such as electrophysiology mapping and electrical ablation. Causes of excess pericardial fluid include extensive intracardiac catheter manipulation and ablation, a need for two or more transseptal punctures, and a need for systemic anticoagulation. Significant PE can lead to hemodynamic decompensation and to potentially life-threatening cardiac tamponade.

Monitoring an undesired impact of catheter procedures on the cardiac anatomy was previously proposed in patent literature. For example, U.S. Patent 9,833,165 describes the monitoring of cardiac ablation to detect hemopericardium by iteratively acquiring magnetic resonance imaging (MRI) data that includes the pericardium, measuring the pericardium by analyzing the sets of MRI data, determining that a measurement of the pericardium in consecutive sets of MRI data differ, and responsively to the determination reporting a change in configuration of the pericardium.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a probe-based ultrasound (US) imaging, electrophysiology mapping and electrical ablation system, in accordance with an example of the present disclosure;
Figs. 2A-2C are schematic ultrasound images of pericardial effusion (PE): mild (Fig. 2A), moderate (Fig. 2B), and severe (Fig. 2C), the ultrasound images obtained by the orientation tracked by US probe of Fig. 1, in accordance with an example of the present disclosure; and
Fig. 3 is a flow chart that schematically illustrates a method to automatically monitor pericardial effusion in real time by analyzing US ultrasound images such as shown in Fig. 2, in accordance with an example of the present disclosure.

### DETAILED DESCRIPTION OF EXAMPLES

### OVERVIEW

During a probe-based clinical procedure in a cardiac chamber, such as electrophysiology mapping or electrical ablation, pericardial effusion (PE) may occur.

To detect PE in a timely manner using conventional means, a user (e.g., a physician) needs to intermittently view cardiac anatomy to check for PE, e.g., by using intracardiac echography (ICE) images, interrupting the workflow of the clinical cardiac procedure. The user, who is typically not an expert in US imaging, is required to stop catheter operations to check PE status visually.

The need to disrupt user workflow may cause late identification of PE. Furthermore, prompt identification may depend on the user's preferences and capability. For example, a user may not have the expertise to obtain ICE images over time that are similar (e.g., similar views) one to another and/or to accurately compare PE levels from the captured images, even if inspected in a timely manner.

Examples of the present disclosure that are described herein provide a technique to detect PE automatically, in real time, and promptly initiate a responsive action by the user, such alerting the user.

A system employed in the disclosed technique monitors, in real time, the status of PE and notifies the user upon crossing a threshold value and/or a significant increase from a baseline value. A position tracking system using an orientation-tracked ICE catheter (and optionally also positioned tracked) allows a system processor to compare images which are acquired from a similar orientation.

In one example, the disclosed technique includes the following steps, of which the critical ones are performed in real time, thereby avoiding any delay in the detection of an adverse event:
- Recording baseline pericardial fluid status during an initial cardiac ICE scan and recording US probe orientation.
- Saving ICE ultrasound image orientation of the baseline recording using the position tracking system.
- Repeatedly acquiring further ultrasound images of the pericardial space in the same orientation (up to a tolerance) to allow for analysis of at least one image in the same ICE ultrasound image orientation as the baseline •
- Monitoring the pericardial space (fluid level) for changes in the image relative to previously taken images. In one example, the processor analyzes pixel intensity changes to estimate the area of fluid in the ultrasound image.
- Notifying the user of PE detection.

In another example, the processor compares a measured width of the pericardial fluid space to a threshold width without a need in a baseline value.

### SYSTEM DESCRIPTION

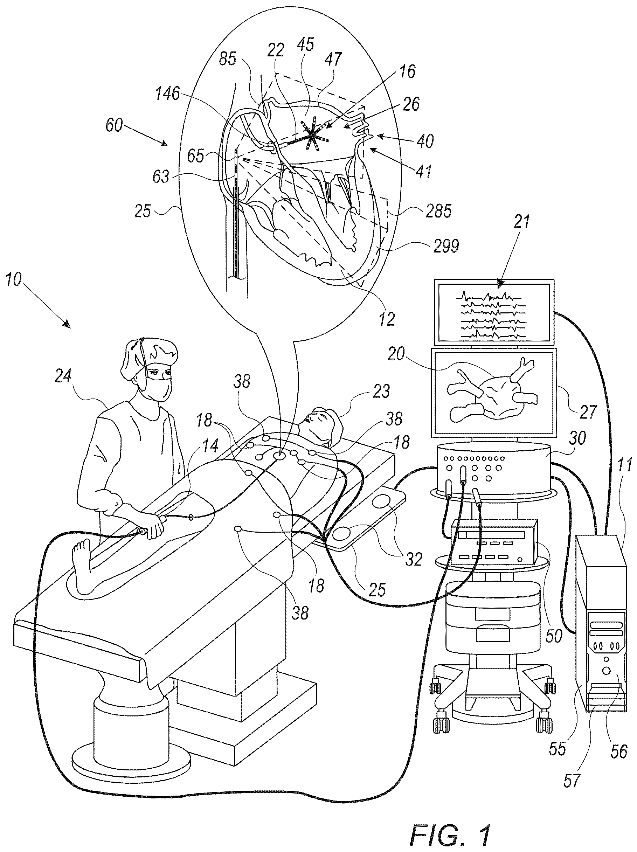

Fig. 1 is a schematic, pictorial illustration of a probe-based ultrasound (US) imaging and electrophysiology mapping and electrical ablation system 10, in accordance with an example of the present disclosure.

System 10 includes a multi-arm catheter 14 percutaneously inserted by physician 24 through the patient's vascular system into a chamber of interest or vascular structure of a heart 12.

As seen in inset 25, physician 24 brings a distal end effector 40 fitted on a shaft 22 of catheter 14 into contact with a target site in heart 12, such as left atrium 45 wall 47 (after effector 40 being inserted via a transeptal puncture 146).

Using electrodes 26 distributed over a plurality of arms 16 at the expandable distal end effector 40, physician 24 operates system 10 to sense intracardiac electrophysiology signals, or to apply electrical ablation, at the target cardiac site.

The physician may, initially or anytime during the procedure, insert an intra-cardiac ultrasound (US) probe 60, comprising a 2D ultrasound array 65 and an integral orientation sensor 63 (optionally sensor 63 is also a position sensor), into heart 12. Optionally and preferably, sensor 63 is a magnetic-based sensor including magnetic coils for sensing orientation and, optionally, three-dimensional (3D) position. In another example, array 65 may be a 1D array that generated a fan-view at a given orientation.

Integral sensor 63 of US probe 21 is preregistered with array 65 of the US probe. Because of the integral location sensor, the spatial coordinates of every voxel in the imaged cardiac chamber are known. Specifically, sensor 63 is configured to output first signals indicative of the location and orientation of the ultrasound transducer array 65 inside heart 26.

During the procedure, console 24 receives orientation signals from sensor 63 in response to magnetic fields from external field generators 32. Magnetic field generators 32 are placed at known positions on a location pad 25 external to patient 23. These orientation signals are indicative of the orientation of ultrasound array 65 in a coordinate system of the position tracking system. When a magnetic sensor includes position capability, the system can track its location in addition to orientation.

Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

As seen in inset 25, the 2D ultrasound array 65 produces a 3D sector-shaped ultrasound beam 85 occupying a defined solid angle, such a beam referred to herein as a "wedge 85." With wedge 85, the 2D ultrasound array can image a substantial volume of an organ, such as an entire cardiac chamber (e.g., the entire left atrium 45).In the disclosed method, electronically tilting the 3D beam, or repositioning probe 60, produces a wedge 285 (or a view 285) that covers a heart 12 cardiac anatomy zone that is prone to accumulation of pericardial space 299.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38, such that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses that may be used to effect irreversible electroporation (IRE), or combinations thereof.

Patient interface unit (PIU) 30 is configured to establish electrical communication between catheters, electrophysiological equipment, power supply and a workstation 55 for controlling operation of system 10. Electrophysiological equipment of system 10 may include, for example, multiple catheters, location pad 25, body surface ECG electrodes 18, electrode patches 38, ablation energy generator 50, and recorder 11. Optionally and preferably, PIU 30 additionally includes processing capability for implementing real-time computations of catheter locations and for performing ECG calculations.

Workstation 55 includes memory 57, processor unit 56 with memory or storage with appropriate operating software loaded therein, and user interface capability. Workstation 55 may provide multiple functions, optionally including (i) modeling endocardial anatomy in three dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of system 10 is available as the CARTO^{™} 3 System, available from Biosense Webster, Inc., 31A Technology Drive, Irvine, CA 92618.

Fig. 1 is brought by way of example. The ICE may be done using a simpler fan-producing ultrasound catheter. Catheter 14 may include a position sensor embedded in or near multi-arm effector 40 to track the position of a distal end 40 of shaft 22.

### INTRA-PROCEDURE AUTOMATIC PERICARDIAL FLUID MONITORING BY ICE

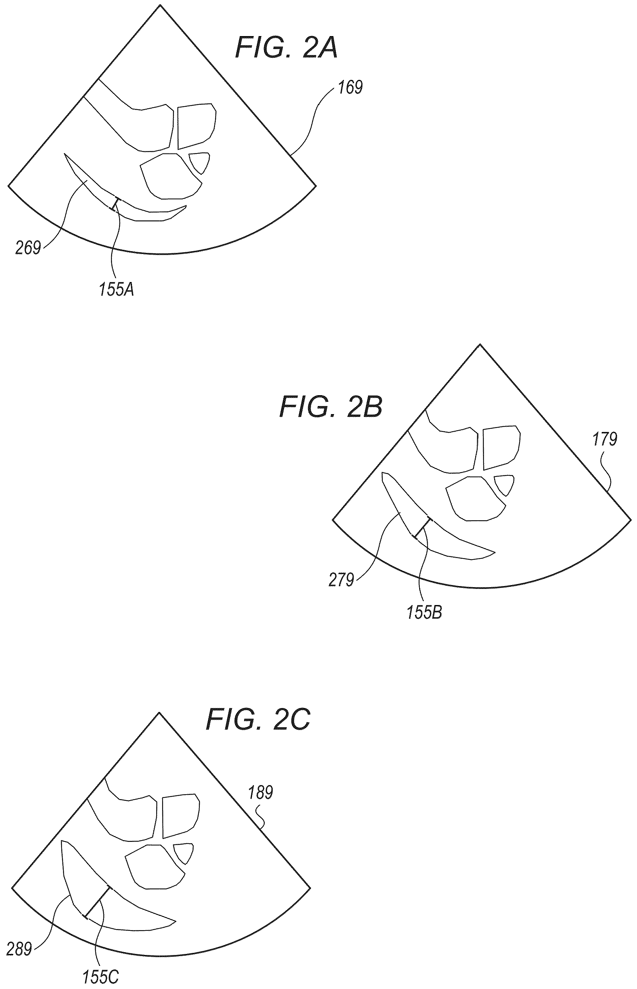

Figs. 2A-2C are schematic ultrasound images (169, 179, 189) of pericardial effusion (PE): mild (Fig. 2A), moderate (Fig. 2B), and severe (Fig. 2C). Ultrasound images (169, 179, 189) are obtained by the orientation of tracked US probe 60 of Fig. 1, in accordance with an example of the present disclosure.

The orientation information about US probe 60 is calculated by processor 56 from signals acquired by magnetic sensor 63, as seen in Fig. 1. This information enables processor 56 to select similar US ultrasound images (169, 179, 189) to assess PE severity during the clinical procedure.

In the example shown, the processor assesses the PE severity by measuring a width 155 of pericardial fluid region (269, 279, 289). The processor alerts the physician if the measured width 155 exceeds a predefined threshold width.

The method shown in Fig. 2 is brought by way of example. In another example, the processor is configured to analyze subsequent ultrasound images (2B-2C) relative to an earlier ultrasound image (2A) by estimating an area occupied by pericardial fluid in an ultrasound image and comparing it to a previously estimated area. The processor is configured to notify the user of PE detection if the area is changed by an amount exceeding a given predefined one, or if the area crosses a predefined threshold area value.

In another example, the processor analyzes a volume of the pericardial fluid in a volumetric US acquisition mode (e.g., in a wedge of images) to monitor PE severity.

### A METHOD FOR INTRA-PROCEDURE AUTOMATIC PERICARDIAL FLUID MONITORING BY ICE

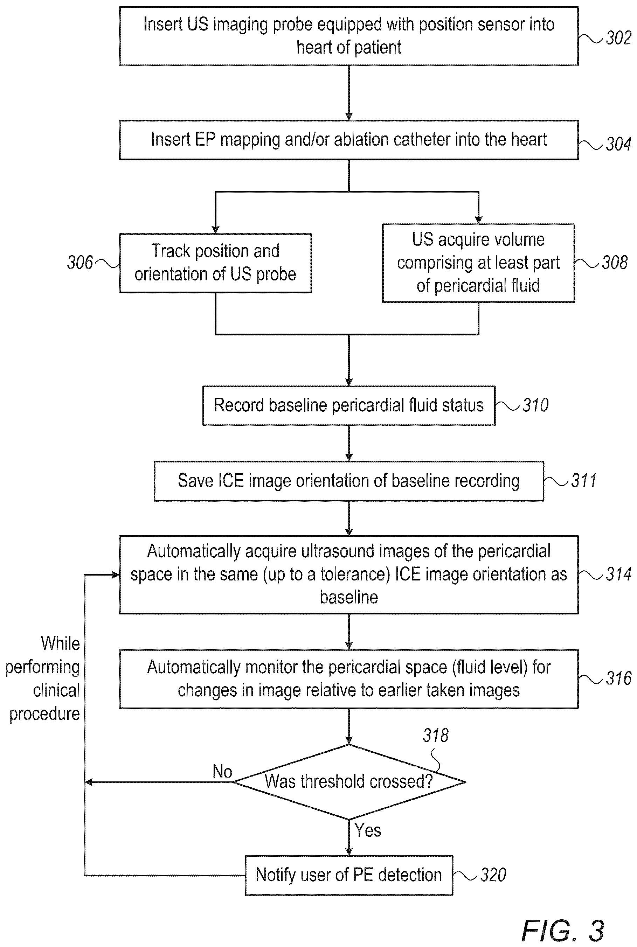

Fig. 3 is a flow chart that schematically illustrates a method to automatically monitor real-time pericardial effusion by analyzing US ultrasound images, such as shown (169, 179, 189) in Fig. 2, in accordance with an example of the present disclosure.

The algorithm, according to the presented example, carries out a process that begins with physician 24 inserting intracardiac echography (ICE) US probe 60 equipped with position sensor 63, inside a cardiac chamber of heart 12, at an US probe insertion step 302.

Next, at a mapping and/or ablation catheter insertion step 304, the physician inserts a distal end effector (e.g., effector 40) into heart 12.

At a tracking step 306, processor 56 tracks the orientation of US probe 60 using sensor 63 on the US probe.

In parallel, at a US acquisition step 308, processor 56 receives an acquired volume wedge 285 from US probe 60 that comprises at least part of the pericardial fluid.

At baseline recording step 310, the system records baseline pericardial fluid status, which is typically expected to be similar, or less, than that seen in Fig. 2A.

The processor records a baseline pericardial fluid status during the initial cardiac ICE scan at baseline recording step 312. This step may involve image analysis to determine an initial value of width 155 of the pericardial fluid.

The processor saves the ICE ultrasound image orientation of the baseline recording, at orientation information saving step 311.

At recurrent acquisition step 314 during the clinical procedure, system 10 automatically acquires further ultrasound images of the pericardial space in the same ICE ultrasound image orientation as the baseline (up to a tolerance).

Processor 56 automatically monitors the pericardial space (fluid level) for changes (e.g., pixel intensity change) in the image relative to earlier images, as seen in Figs 2A-2C, at a monitoring step 316.

At threshold crossing checking step 318, the processor checks if the threshold was crossed. If not, the process returns to step 314 to acquire new data.

If width 155 is found to exceed the threshold value, processor 56 notifies the user of PE detection, such as by activating an audiovisual alert, at notification step 320. The monitoring process continues by returning to step 314.

The flow chart shown in Fig. 3 is chosen purely for conceptual clarity. The present example may be applied, with the necessary changes made, with any type of US probe that includes a way to track its orientation.

### EXAMPLES

### Example 1

A medical system (10) includes an ultrasound probe (60) and a processor (56). The ultrasound probe is configured for insertion into the heart (12) of a body and comprises (a) an ultrasound transducer array (65) configured to generate ultrasound images (169, 179, 189) of a portion of the heart and (b) a sensor (63) configured to produce signals indicative of an orientation (285) of the ultrasound transducer array (65) inside the heart. The processor is configured to (i) select, using the signals produced by the sensor (63), a sequence of two or more of the ultrasound images (169, 179, 189) that are acquired from a given orientation (285) of the ultrasound transducer array (65), wherein the ultrasound images (169, 179, 189) image at least part of a pericardial space (299) of the heart, (ii) estimate changes in Pericardial Effusion (PE) in the pericardial space (299), by analyzing the selected sequence of the ultrasound images (169, 179, 189), and (iii) initiate a responsive action when the changes meet a defined condition.

### Example 2

The system (10) according to example 1, wherein the processor (56) is configured to estimate changes in PE by recording (310) a baseline PE status, during an initial cardiac ultrasound probe (60) scan, and comparing PE level in subsequent images to the baseline.

### Example 3

The system (10) according to any of examples 1 and 2, wherein the processor (56) is configured to estimate changes in PE by defining area borders to monitor (316) for the changes in PE and estimate visual changes in the area.

### Example 4

The system (10) according to any of examples 1 and 2, wherein the processor (56) is configured to estimate changes in PE by estimating a width (155A, 155B, 155C) occupied by pericardial fluid and comparing it to previously estimated width.

### Example 5

The system (10) according to any of examples 1, 2, and 4, wherein the processor (56) is further configured to notify the user of PE detection when the width (155A, 155B, 155C) crosses a predefined threshold width value.

### Example 6

The system (10) according to any of examples 1 and 2, wherein the processor (56) is configured to estimate changes in PE by estimating an area occupied by pericardial fluid in the ultrasound image (169, 179, 189) and comparing it to a previously estimated area.

### Example 7

The system (10) according to any of examples 1 and 2, and 6, wherein the processor (56) is further configured to notify the user of PE detection when the area changes by an amount above a predefined threshold area value.

### Example 8

The system (10) according to any of examples 1 through 7, wherein the sensor (63) is configured to generate the signals in response to a magnetic field applied by a position tracking system.

### Example 9

The system (10) according to any of examples 1 through 8, wherein the processor (56) is configured to select the ultrasound image (169, 179, 189) by using an image processing algorithm.

### Example 10

The system (10) according to any of examples 1 through 9, wherein the processor (56) is configured to initiate a responsive action by alerting the user of PE detection.

### Example 11

A method includes inserting an ultrasound probe (60) into a heart (12) of a body, the ultrasound probe comprising (a) an ultrasound transducer array (65) configured to generate ultrasound images (169, 179, 189) of a portion of the heart, and (b) a sensor (63) configured to produce signals indicative of an orientation (285) of the ultrasound transducer array (65) inside the heart. Using the signals produced by the sensor, a sequence of two or more of the ultrasound images (169, 179, 189) is selected that are acquired from a given orientation (285) of the ultrasound transducer array (65), wherein the ultrasound images (169, 179, 189) image at least part of a pericardial space (299) of the heart. Changes are estimated in Pericardial Effusion (PE) in the pericardial space (299), by analyzing the selected sequence of the ultrasound images (169, 179, 189). A responsive action is initiated when the changes meet a defined condition.

### Example 12

The method according to example 11, wherein estimating changes in PE comprises recording a baseline PE status, during an initial cardiac ultrasound probe scan, and comparing PE level in subsequent images to the baseline.

### Example 13

The method according to example 11, wherein estimating changes in PE comprises defining area borders to monitor for the changes in PE and estimate visual changes in the area.

### Example 14

The method according to example 11, wherein estimating changes in PE comprises estimating a width occupied by pericardial fluid and comparing it to previously estimated width.

### Example 15

The method according to example 14, and comprising notifying the user of PE detection when the width crosses a predefined threshold width value.

### Example 16

The method according to example 11, wherein estimating changes in PE comprises estimating an area occupied by pericardial fluid in the ultrasound image and comparing it to a previously estimated area.

### Example 17

The method according to example 16, wherein the processor is further configured to notify the user of PE detection when the area changes by an amount above a predefined threshold area value.

### Example 18

The method according to example 11, wherein the sensor is configured to generate the signals in response to a magnetic field applied by a position tracking system.

### Example 19

The method according to example 11, wherein selecting the ultrasound image comprises using an image processing algorithm.

### Example 20

The method according to example 11, wherein initiating a responsive action comprises alerting the user of PE detection.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and sub combinations of the various features described hereinabove, as well as variations and modifications thereof which would occur to persons skilled in the art upon reading the foregoing description and which are not disclosed in the prior art.

## Claims

1. A medical system (10), comprising:
an ultrasound probe (60) for insertion into a heart (12) of a body, the ultrasound probe comprising:
an ultrasound transducer array (65) configured to generate ultrasound images (169, 179, 189) of a portion of the heart; and
a sensor (63) configured to produce signals indicative of an orientation (285) of the ultrasound transducer array (65) inside the heart; and
a processor (56), which is configured to:
select, using the signals produced by the sensor (63), a sequence of two or more of the ultrasound images (169, 179, 189) that are acquired from a given orientation (285) of the ultrasound transducer array (65), wherein the ultrasound images (169, 179, 189) image at least part of a pericardial space (299) of the heart;
estimate changes in Pericardial Effusion (PE) in the pericardial space (299), by analyzing the selected sequence of the ultrasound images (169, 179, 189); and
initiate a responsive action when the changes meet a defined condition.

2. The system (10) according to claim 1, wherein the processor (56) is configured to estimate changes in PE by recording (310) a baseline PE status, during an initial cardiac ultrasound probe scan, and comparing PE level in subsequent images to the baseline.

3. The system (10) according to any of claims 1 and 2, wherein the processor (56) is configured to estimate changes in PE by defining area borders to monitor (316) for the changes in PE and estimate visual changes in the area.

4. The system (10) according to any of claims 1 and 2, wherein the processor (56) is configured to estimate changes in PE by estimating a width (155A, 155B, 155C) occupied by pericardial fluid and comparing it to previously estimated width.

5. The system (10) according to any of claims 1, 2, and 4, wherein the processor (56) is further configured to notify the user of PE detection when the width (155A, 155B, 155C) crosses a predefined threshold width value.

6. The system (10) according to any of claims 1 and 2, wherein the processor (56) is configured to estimate changes in PE by estimating an area occupied by pericardial fluid in the ultrasound image (169, 179, 189) and comparing it to a previously estimated area.

7. The system (10) according to claims 1, 2, and 6, wherein the processor (56) is further configured to notify the user of PE detection when the area changes by an amount above a predefined threshold area value.

8. The system (10) according to any of claims 1 through 7, wherein the sensor (63) is configured to generate the signals in response to a magnetic field applied by a position tracking system.

9. The system (10) according to any of claims 1 through 8, wherein the processor (56) is configured to select the ultrasound image (169, 179, 189) by using an image processing algorithm.

10. The system (10) according to any of claims 1 through 9, wherein the processor (56) is configured to initiate a responsive action by alerting the user of PE detection.

11. A method, comprising:
inserting an ultrasound probe (60) into a heart (12) of a body, the ultrasound probe comprising:
an ultrasound transducer array (65) configured to generate ultrasound images (169, 179, 189) of a portion of the heart; and
a sensor (63) configured to produce signals indicative of an orientation (285) of the ultrasound transducer array (65) inside the heart.
selecting, using the signals produced by the sensor, a sequence of two or more of the ultrasound images (169, 179, 189) that are acquired from a given orientation (285) of the ultrasound transducer array (65), wherein the ultrasound images (169, 179, 189) image at least part of a pericardial space (299) of the heart;
estimating changes in Pericardial Effusion (PE) in the pericardial space (299), by analyzing the selected sequence of the ultrasound images; and
initiating a responsive action when the changes meet a defined condition.
